# EUROPÄISCHE PATENTSCHRIFT

(11) **EP 0 895 983 B1**
(45) Veröffentlichungstag und Bekanntmachung des Hinweises auf die Patenterteilung: **03.04.2002**
(21) Anmeldenummer: 98113847.2
(22) Anmeldetag: 24.07.1998
(51) Int. Cl.: C07C 209/36, C07C 209/38, C07C 211/55

(54) **Verfahren zur Herstellung von 4-Aminodiphenylamin**
Process for the preparation of 4-aminodiphenylamine
Procédé pour la préparation de la 4-aminodiphenylamine

(30) Priorität: 06.08.1997 DE 19734055
(43) Veröffentlichungstag der Anmeldung: 10.02.1999
(73) Patentinhaber: BAYER AG, 51368 Leverkusen (DE)
(72) Erfinder: Ooms, Pieter, Dr., 47800 Krefeld (DE); Notheis, Ulrich, Dr., 41539 Dormagen (DE); Laue, Christian, Dr., 40789 Monheim (DE)

(56) Entgegenhaltungen:
- EP-A- 0 784 049

## Beschreibung

Die Erfindung betrifft ein Verfahren zur Herstellung von 4-Aminodiphenylamin (4-ADPA) durch Hydrierung von Nitrosobenzol mit Wasserstoff in Gegenwart von Hydrierkatalysatoren und Basen.

4-ADPA ist ein wichtiges Zwischenprodukt für Alterungsschutzmittel und Stabilisatoren in der Gummi- und Polymerindustrie (Kirk-Othmer, Encyclopedia of Chemical Technology, 4th Edition, 1992, Vol. 3, S. 424-447 und S 448-456; Ullmann's Encyclopedia of Industrial Chemistry, 5th Edition, Vol A3, 1985, S 91-111).

4-Aminodiphenylamin kann nach verschiedenen Methoden hergestellt werden. Eine Möglichkeit 4-ADPA herzustellen, ist die zweistufige (Zwischenprodukt 4-Nitrodiphenylamin) Umsetzung von Anilin bzw. Anilinderivaten mit p-Nitrochlorbenzol in Gegenwart eines Säureakzeptors oder eines Neutralisierungsmittels und gegebenenfalls in Gegenwart eines Katalysators. Die Herstellung nach dieser Methode ist beispielsweise beschrieben in DE-A 35 01 698, DE-A 18 56 63, US 4 670 595, US 4 187 249, US 468 333 und US 4 187 248. Ein Nachteil eines solchen Verfahrens ist, daß die dabei entstehenden Halogenidionen zu Korrosion in Reaktoren und Apparaturen führen und mit beträchtlichen Kosten entsorgt werden müssen. Daher hat man Anilin bzw. entsprechende Anilinderivate mit Nitrobenzol in Gegenwart von Tetraalkylammoniumhydroxiden und in Gegenwart kontrollierter Mengen an protischen Materialien umgesetzt. 4-ADPA wurde dabei in einer befriedigenden Menge erhalten (siehe WO 95/00 324 und WO 93 24 450). Nach US 5 420 354 kann man 4-ADPA durch Umsetzung von Anilin, Nitrobenzol und Wasserstoff in Gegenwart eines Hydrierkatalysators, Hydrierinhibitors und sauren Co-Katalysators allerdings in wenig befriedigenden Ausbeuten erhalten. In US 5 574 187 wird beschrieben, daß man durch Umsetzung von Anilin mit Nitrosobenzol oder Phenylhydroxylamin in Gegenwart von Säuren 4-ADPA erhalten kann.

Diese Verfahren haben jedoch den Nachteil, daß zwei verschiedene Edukte, die in vorgelagerten separaten Verfahrensschritten hergestellt werden müssen, eingesetzt werden, was weniger wirtschaftlich ist.

Es ist auch bekannt, daß die Hydrierung von Nitrosobenzol über heterogene Katalysatoren hauptsächlich Anilin und Hydrazobenzol liefert. 4-ADPA wird als Produkt nicht erwähnt (Chem. Ind. 1994, Catalysis of Organic Reactions, S. 137-149).

*Aus EP-A 784049 ist ein Verfahren zur Herstellung von 4-Aminodiphenylamin bekannt, indem man ggf. substituiertes Anilin mit ggf. substituertem Nitrobenzol in Gegenwart von Wasser und/oder Alkoholen und organischen und/oder anorganischen Basen umsetzt und anschließend das erhaltene Nitro- und/oder Nitrosodiphenylamin katalytisch in Gegenwart von Wasser hydriert, wobei man die katalytische Hydrierung des Reaktionsgemischs in Gegenwart von 25 bis 80 Gew.-% Wasser, bezogen auf das Gewicht des Reaktionsgemisches aus der Kondensationsreaktion, durchführt, nach Beendigung der Wasserstoffaufnahme das Hydriergemisch vom Hydrierkatalysator befreit, ggf. dem Hydriergemsch 10 bis 100 Vol.-% aromatisches Lösungsmittel, bezogen auf das Gesamtvolumen des Hydriergemischs, zugibt, die gebildete organische Phase zur Isolierung des 4-Aminodiphenylamins abtrennt und die wäßrige Phase dem Ausgangsreaktionsgemisch wieder zuführt.*

Überraschenderweise wurde nun gefunden, daß man durch Hydrierung von Nitrosobenzol in Gegenwart von Basen und heterogenen Katalysatoren 4-ADPA in technisch brauchbaren Ausbeuten erhalten kann.

Gegenstand der vorliegenden Erfindung ist daher ein Verfahren zur Herstellung von 4-Aminodiphenylamin, das dadurch gekennzeichnet ist, daß man Nitrosobenzol oder Gemische aus Nitrosobenzol und Nitrobenzol mit Wasserstoff in Gegenwart von hydroxid-, oxid- und/oder alkoxidgruppenhaltigen Basen und *in Gegenwart von Metallen der 8. - 10. Gruppe des Periodensystems (nach IUPAC, neu) oder Kupfer und/oder Chrom, ggf*. *aufgebracht auf einen Katalysatorträger,* sowie in Gegenwart von inerten aprotischen Lösungsmitteln bei Temperaturen von 0 bis 200°C und Drücken von 0,1 bis 150 bar hydriert.

Als für das erfindungsgemäße Verfahren geeignete hydroxid-, oxid- und/oder alkoxidgruppenhaltige Basen kommen in Frage anorganische Basen, wie Alkalimetallhydroxide, Alkalimetalloxide, Alkalimetallalkoxide, Erdalkalimetallhydroxide, Erdalkalimetalloxide, Erdalkalimetallalkoxide sowie die entsprechenden Hydroxide, Oxide und Alkoxide der Elemente 58 bis 71 des Periodensystems der Elemente (nach IUPAC, neu). Beispielsweise werden genannt: Die Oxide, Hydroxide und Alkoxide von Natrium, Kalium, Lithium, Caesium, Magnesium, Calcium, Barium, Lanthan und/oder Cer, insbesondere die Oxide, Hydroxide und Alkoxide von Lithium, Natrium, Kalium, Caesium, ganz besonders bevorzugt Kaliumhydroxid, Caesiumhydroxid, Natriummethylat, Natriumethylat, Natriumbutylat, Kaliumbutylat, Kaliumpropylat, Kaliummethylat, Kaliumethylat, Kaliumpentylat.

Weiterhin kommen organische Basen in Frage, wie beispielsweise quatemäre Alkylammoniumhydroxide (NR₄⁺OH⁻ mit R unabhängig voneinander für Alkyl, Aryl oder Aralkyl mit 1 bis 7 Kohlenstoffatomen). Als Beispiele seien genannt: Tetramethylammoniumhydroxid, Tetraethylammoniumhydroxid, Tetrapropylammoniumhydroxid, Tetrabutylammoniumhydroxid, Methyltributylammoniumhydroxid, Methyltripropylammoniumhydroxid, Methyltriethylammoniumhydroxid, Trimethylbenzylammoniumhydroxid. Besonders bevorzugt sind Tetramethylammoniumhydroxid, Tetraethylammoniumhydroxid, Tetrapropylammoniumhydroxid und Tetrabutylammoniumhydroxid. Ganz besonders bevorzugt wird Tetramethylammoniumhydroxid eingesetzt.

Es können auch beliebige Mischungen der vorgenannten Basen eingesetzt werden.

Weiterhin ist es möglich, die anorganischen Basen in Verbindung mit Phasentransferkatalysatoren einzusetzen. Geeignete Phasentransferkatalysatoren sind beispielsweise beschrieben in W.E. Keller, Fluka-Kompendium, Bd. 1, 2, 3, Georg Thieme Verlag, Stuttgart 1986, 1987, 1992. Beispielsweise können die zuvor erwähnten Basen mit Kronenether, wie 18-Krone-6 oder quaternäre Ammoniumverbindungen, zusammen eingesetzt werden.

Die erfindungsgemäß einzusetzenden Basen können einen Wassergehalt von bis zu 6 mol Wasser, bevorzugt bis zu 3 mol Wasser, besonders bevorzugt bis zu 2,5 mol Wasser, bezogen auf ein Mol Base, besitzen.

Die erfindungsgemäßen Basen können dem Reaktionsgemisch in fester Form, als Schmelze oder als Lösung in einem aprotischen Lösungsmittel oder in einer Mischung aus Nitrosobenzol und einem oder mehreren aprotischen Lösungsmitteln zugesetzt werden.

Die Basen werden dabei in einer Menge von 0,01 bis 3, bevorzugt 0,1 bis 2, insbesondere 0,3 bis 1,5 Äquivalenten pro Mol Nitrosobenzol eingesetzt.

Als inerte aprotische Lösungsmittel kommen aromatische Kohlenwasserstoffe mit 6 bis 20 Kohlenstoffatomen, lineare oder cyclische Ether mit bis zu 5 Sauerstoffatomen und 2 bis 16 Kohlenstoffatomen, aromatische halogenierte Kohlenwasserstoffe mit 6 bis 20 Kohlenstoffatomen sowie Amide mit 1 bis 10 Kohlenstoffatomen in Frage. Selbstverständlich können die erwähnten Lösungsmittel im Gemisch untereinander eingesetzt werden. Als geeignete Lösungsmittel werden insbesondere genannt: Benzol, Toluol, Xylol, tert.-Butylmethylether, tert.-Amylmethylether, Diisopropylether, Diethylenglykoldimethylether, Glycoldimethylether, Dioxan, Tetrahydrofuran, Diamylether, Chlorbenzol, Dichlorbenzol, Dimethylformamid, Dimethylacetamid und N-Methylpyrolidinon. Bevorzugt werden eingesetzt, Toluol, Xylol, Glykoldimethylether, Dioxan, tert.-Butylmethylether, Diisopropylether, Diethylenglykoldimethylether, insbesondere Glykoldimethylether, Dioxan, tert.-Butylmethylether, Diethylenglykoldimethylether und Toluol. Die Menge an Lösungsmittel ist für das erfindungsgemäße Verfahren nicht kritisch und hängt insbesondere ab von der Reaktionstemperatur und von der Art und Menge der eingesetzten Basen und Katalysatoren. Üblicherweise werden die Lösungsmittel in Mengen von 1 bis 99 Gew.-%, bevorzugt 5 bis 95 Gew.-%, besonders bevorzugt 15 bis 90 Gew.-%, bezogen auf die Gesamtmenge der Reaktionsmischung, eingesetzt.

*Die erfindungsgemäß einzusetzenden heterogenen Katalysatoren umfassen* Metalle der 8-10 Gruppe des Periodensystems (nach IUPAC, neu) oder Kupfer und/oder Chrom auf geeignetem Träger mit einem Metallgehalt von 0,01 bis 50 Gew.-%, bevorzugt 0,1 bis 10 Gew.-%, bezogen auf das Gesamtgewicht des Katalysators. Erfindungsgemäß können Katalysatoren eingesetzt werden, die eines oder mehrere der obengenannten Metalle enthalten. Die angegebenen Gewichtsanteile gelten bei Anwesenheit mehrerer Elemente für die Summe der Einzelanteile. Bevorzugte Metalle sind insbesondere Platin, Palladium, Rhodium und Ruthenium, besonders bevorzugt sind Platin, Palladium und Rhodium. Weitere bevorzugte Katalysatoren sind Raney-Nickel und geträgerte Nickelkatalysatoren.

Erfindungsgemäß können auch die obengenannten Metalle oder ihre Verbindungen in reiner Form als Feststoff eingesetzt werden. Als Beispiele für ein Metall in reiner Form seien Palladium- und Platinschwarz genannt.

Die Herstellung der erfindungsgemäß einzusetzenden Katalysatoren kann nach den verschiedensten Methoden erfolgen, die dem Fachmann bekannt sind. So können Lösungen einer oder mehrerer der genannten Metallverbindungen, beispielsweise durch Tränken, Adsorption, Tauchen, Sprühen, Imprägnieren und Ionenaustausch auf den erfindungsgemäß einzusetzenden Katalysatorträger gebracht werden. Dem Katalysator können in bekannter Art und Weise weitere Elemente zugefügt werden. Es ist weiterhin möglich, ein oder mehrere der genannten Metalle durch Fällung mit einer Base auf dem Träger zu fixieren. Als Base kommen z.B. (Erd-)Alkalimetallhydroxide in Frage. Ein oder mehrere Metalle können sowohl in beliebiger Reihenfolge nacheinander als auch gleichzeitig auf den Träger gebracht werden. Eine spezielle Ausführungsform der Erfindung beinhaltet das Aufbringen des Metalls durch Fällung eines Metallhalogenids oder einer Metallhalogenid-Komplexverbindung mit einer geeigneten Base und Reduktion der Metallverbindung zum Metall. Bei der Herstellung der Träger mittels eines Sol-Gel-Verfahrens können in einer Ausführungsform Lösungen einer oder mehrerer der genannten Metallverbindungen in dem Fachmann bekannter Weise bereits dem Sol zugesetzt werden.

Geeignete Materialien für die erfindungsgemäße Verwendung als Katalysatorträger sind alle technisch üblichen Katalysatorträger auf der Basis Kohlenstoff, Elementoxiden, Elementcarbiden oder Elementsalzen in verschiedenen Anwendungsformen. Beispiele für kohlenstoffhaltige Träger sind Koks, Graphit, Ruß oder Aktivkohlen. Beispiele für Elementoxid-Katalysatorträger sind SiO₂ (natürliche oder synthetische Kieselsäure, Quarz), Al₂O₃ (α-, γ-Al₂O₃), Tonerden, natürliche und synthetische Alumosilicate (Zeolithe), Schichtsilikate, wie Bentonit und Montmorillonit, TiO₂ (Rutil, Anatas), ZrO₂, MgO oder ZnO. Beispiele für Elementcarbide und -salze sind SiC, AlPO₄, BaSO₄, CaCO₃. Grundsätzlich können sowohl synthetische Materialien als auch Träger aus natürlichen Quellen, wie z.B. Bimsstein, Kaolin, Bleicherden, Bauxite, Bentonite, Kieselgur, Asbest oder Zeolithe, verwendet werden.

Weitere brauchbare Träger für die erfindungsgemäß einsetzbaren Katalysatoren sind Elementmischoxide und Oxidhydrate von Elementen der Gruppen 2 bis 16 des Periodensystems sowie der Seltenerdmetalle (Atomnummern 58 bis 71), bevorzugt aus den Elementen Al, Si, Ti, Zr, Zn, Mg, Ca, Zn, Nb und Ce, die u.a. auf dem Weg über mechanische Vermischungen, gemeinsame Fällungen von Salzen oder über Cogele aus Salzen und/oder Alkoxiden hergestellt werden können, wie dies dem Fachmann bekannt ist.

Beispiele für Mischoxide sind Magnesiumaluminiumoxide (Hydrotalcite).

Die Träger können sowohl im Sinne chemisch einheitlicher Reinsubstanzen als auch im Gemisch eingesetzt werden. Für die erfindungsgemäße Verwendung als Katalysatorträger eignen sich sowohl stückige als auch pulverförmige Materialien. Für den Fall der Anordnung des Träger-Katalysators als Festbett wird der Träger vorzugsweise als Formkörper, z.B. als Kugeln, Zylinder, Stäbchen, Hohlzylinder, Ringe usw. eingesetzt. Wahlweise können Katalysatorträger weiter durch Extrudieren, Tablettieren, gegebenenfalls unter Zumischen weiterer Katalysatorträger oder Bindemittel, wie SiO₂ oder Al₂O₃, und Kalzinieren modifiziert werden. Die innere Oberfläche der Träger (BET-Oberfläche) liegt bei 1 bis 2 000 m²/g, bevorzugt bei 10 bis 1 600 m²/g, ganz besonders bevorzugt bei 20 bis 1 500 m²/g. Darstellung und Weiterverarbeitung der erfindungsgemäß verwendeten Katalysatorträger sind dem Fachmann wohl bekannt und Stand der Technik.

Bevorzugt werden Aktivkohlen und Si-, Al-, Mg-, Zr- und Ti-haltige Materialien als Trägermaterialien eingesetzt, besonders bevorzugt sind Aktivkohle sowie silizium-, magnesium- und aluminiumhaltige Träger.

Die erfindungsgemäß verwendeten Katalysatoren können in diskontinuierlichen Verfahrensvarianten in Mengen von 0,01 bis 20 Gew.-% bezogen auf eingesetztes Nitrosobenzol verwendet werden, bevorzugt in Mengen von 0,01 bis 10 Gew.-%. Bei kontinuierlicher Durchführung der Reaktion, beispielsweise in einem Rührkessel mit einem pulverförmigen Katalysator oder in der Rieselphase am Festbettkatalysator, können Belastungen von 0,01 bis 500 g Nitrosobenzol pro g Katalysator und Stunde verwendet werden. Bevorzugt sind Belastungen von 0,02 bis 300 g Nitrosobenzol pro g Katalysator und Stunde.

In das erfindungsgemäße Verfahren können Nitrosobenzol oder Nitrosobenzol/Nitrobenzolgemische, wie sie beispielsweise bei der Herstellung von Nitrosobenzol aus Nitrobenzol anfallen, eingesetzt werden. Der Gehalt an Nitrosobenzol kann beispielsweise 0,5 bis 99 %, bevorzugt 0,5 bis 98 %, besonders bevorzugt 1 bis 97 % sein.

Die Reaktionstemperaturen bei dem erfindungsgemäßen Verfahren betragen bevorzugt 0 bis 200°C, insbesondere 25 bis 150°C; die Drücke (Wasserstoffdruck) liegen bei 0,1 bis 150 bar, insbesondere 0,5 bis 70 bar, ganz besonders bevorzugt 1 bis 50 bar.

Es ist möglich, die Reaktion bei einer konstanten Temperatur und bei konstantem Wasserstoffdruck durchzuführen; Wasserstoffdruck und Temperatur können aber auch im Verlauf der Reaktion geändert werden bzw. in verschiedenen Reaktoren verschieden sein. Bei der diskontinuierlichen Durchführung können Nitrosobenzol, Katalysator, Lösungsmittel und Base in beliebiger Reihenfolge in den Reaktor eingespeist werden. Die Wasserstoffzufuhr kann nach einer bestimmten zugeführten Menge abgebrochen und gegebenenfalls später wieder fortgesetzt werden.

Kontinuierliche Verfahrensvarianten sind beispielsweise die Hydrierung in der Sumpfphase mit einem pulverförmigen suspendierten Katalysator (Slurry), die Hydrierung in der Rieselphase am Festbett-Katalysator oder die Hydrierung mit einem suspendierten Katalysator in einer Blasensäule. Die Reaktion kann in den dem Fachmann bekannten Apparaten zur Kontaktierung von Fest-, Flüssig- und Gasphasen durchgeführt werden. Insbesondere kommen hier Rührkessel, Umpumpreaktoren, Busreaktoren, im Gleich- oder Gegenstrom betriebene Blasensäulen oder Rieselphasenreaktoren oder Kaskaden dieser Reaktoren, in Frage, wobei die verschiedenen Reaktortypen auch gleichzeitig in einer Kaskade vorkommen können.

Wird der Katalysator als Pulver in der Sumpfphase eingesetzt, sind zur Vermischung der Reaktionskomponenten, die zu verwendenden Rührbehälter mit dafür brauchbaren Rührern ausgestattet. Möglich sind der Einsatz von Flügel-, MIG-, Propeller-, Anker- oder Begasungsrührer.

Die darüber hinaus bei dem erfindungsgemäßen Verfahren anfallenden Stoffe sind Zwischenprodukte der Hydrierung von Nitrosobenzol zu Anilin und lassen sich restlos in Anilin überführen, welches ebenfalls ein wertvolles Ausgangsprodukt für die Synthese vieler industrieller Endprodukte ist.

### Beispiele

Folgende Beispiele demonstrieren die Durchführbarkeit der Reaktion bei verschiedenen Drücken und Temperaturen und unter Verwendung verschiedener Katalysatoren, Basen und Lösungsmittel. Die Reaktionsprodukte wurden gas-chromatografisch (Durabond DB-5-MS; 30 m x 0,25 mm ID) mit dem internen Standard n-Tridekan bzw. mittels quantitativer HPLC analysiert. Der Umsatz an Nitrosobenzol war in allen beschriebenen Versuchen vollständig. Aufarbeitung und Probenpräparation wurde unter Stickstoff durchgeführt. Die angegebenen Hydrierdrücke im Autoklav wurden bei reaktionsbedingtem Druckabfall von Hand nachgeregelt.

Die eingesetzten Basen und Katalysatoren sind käufliche Produkte oder wurden wie im folgenden beschrieben, hergestellt.

Die Tetramethylammoniumhydroxid-Hydrate (TMAOH. x H₂O) wurden durch Wasserentzug eines handelsüblichen Pentahydrats (Fa. Aldrich) oder einer 25 %-igen wäßrigen Lösung (Fa. Aldrich) bei 80°C im Wasserstrahlvakuum und danach im Pumpenvakuum oder im Exsiccator über Phosphorpentoxid hergestellt. Der verbliebene Wassergehalt wurde durch Säure-Titration festgestellt. Der Wassergehalt des jeweils eingesetzten Materials ist in den Ansätzen spezifiziert.

### Herstellung der Katalysatoren A, B

### Katalysator A (Pd, Rh und Pt-Katalysatoren)

Das Trägermaterial wurde 2 h bei 350°C im Muffelofen ausgeheizt und in einen 250 ml Kolben überführt. Am Rotationsverdampfer wurde zum Trägermaterial eine verdünnte wäßrige Lösung von PdCl₂, RhCl₃ oder H₂PtCl₆ (durch Verdünnen einer wäßrigen H₂PtCl₆-Lösung mit 25 % Platingehalt hergestellt) gegeben. Die Wassermenge war so gewählt worden, daß sie komplett vom Träger aufgenommen wurde ("incipient wetnet"). Anschließend wurde der größte Teil des Wassers bei 60°C im Vakuum abdestilliert, der Katalysator 16 h bei 120°C bei einem Druck <1 mbar getrocknet und in einem Strom von 10 Vol-% Wasserstoff in Stickstoff für 24 h bei 300°C reduziert. Der Edelmetall-Gehalt wurde mittels Elementaranalyse bestimmt.

| Bsp.Nr | Typ | Träger | BET m²/g | Träger (g) | "M" | "H₂O" | "M%" |
|---|---|---|---|---|---|---|---|
| A1 | Pd/MgO | Aldrich | 46 | 20,0 | 1,7 | 400 | 5,1 |
| A2 | Rh/HT | Hydrotalcit Südchemie | 134 | 15,0 | 2,0 | 60 | 3,9 |
| A3 | Pt/HT | Hydrotalcit Südchemie | 93 | 23,8 | 5,0 | 90 | 3,8 |
| "M": Menge an PdCl2, RhCl3 oder H₂PtCl₆-Lösung (25% Pt-Gehalt); "H₂O": Menge an zugegebenem Wasser; (im Falle von PdCl2 wird ein Tropfen konz. HCl zugegeben) | | | | | | | |
| M%: Edelmetallgehalt des fertigen Katalysators in Gew.-%. | | | | | | | |

### Katalysator B (Pt/Aktivkohle)

475 g Aktivkohle (Norit-B-Supra, Fa. Norit) wurden in 2 600 ml entionisiertem Wasser aufgeschlämmt, die Mischung auf 50°C erwärmt und mit einer Lösung von 87,5 g Natriumformiat in 400 ml entionisiertem Wasser versetzt. In 30 Minuten wurde eine Mischung aus 100 g einer H₂PtCl₄-Lösung (25 Gew.-% Pt) und 400 ml entionisiertem Wasser zugetropft und eine Stunde bei 50°C nachgerührt. Anschließend wurde der Katalysator abgesaugt, gewaschen und im Vakuum bei 60°C getrocknet.

### Beispiele 1 bis 22 (Hydrierungen unter Normaldruck)

### Beispiel 1

In einem mit Stickstoff gespülten 250 ml Planschlifftopf mit Begasungsrührer wurden 65 ml Diethylenglykoldimethylether, 7,03 g (0,055 mol) TMAOH. 2,0 H₂O und 0,5 g eines pulverförmigen 5 % Pd/C-Katalysator 3230 der Firma Engelhard vorgelegt und auf 80°C erwärmt. Nach Erreichen dieser Temperatur wurde der Stickstoff bei Normaldruck durch einen Wasserstoffstrom von 25 l/h ersetzt und gleichzeitig 5,89 g (0,055 mol) Nitrosobenzol in 10 ml Diethylenglykoldimethylether zugegeben. Nach 120 min. wurde eine Probe entnommen, filtriert, mit Essigsäure neutralisiert und mittels quantitativer Gaschromatografie analysiert. Der Umsatz an Nitrosobenzol war vollständig. Die Ausbeute an 4-ADPA betrug 46,0 % (Die %-Angaben sind mol-% bezogen auf Nitrosobenzol).

### Beispiel 2

Das Beispiel 1 wurde mit 0,5 g eines pulverförmigen 5 % Pt/C-Katalysators (Katalysator B) bei 80°C wiederholt. Nach 2 h Reaktionszeit betrug die Ausbeute an 4-ADPA 48,0 %.

### Beispiel 3

Das Beispiel wurde mit 0,5 g eines pulverförmigen 5 % Rh/C-Katalysators G 10 der Firma Degussa bei 80°C wiederholt. Nach 2 h Reaktionszeit betrug die Ausbeute an 4-ADPA 47,0 %.

### Beispiel 4

Das Beispiel 1 wurde mit 0,5 g eines pulverförmigen 5 % Pt/Al₂O₃-Katalysators der Firma Aldrich bei 80°C wiederholt. Nach 2 h Reaktionszeit betrug die Ausbeute an 4-ADPA 35,3 %.

### Beispiel 5

Das Beispiel 1 wurde mit 2,5 g eines pulverförmigen 1 % Pt/ZSM 5-Katalysators bei 80°C wiederholt. Nach 2 h Reaktionszeit betrug die Ausbeute an 4-ADPA 31,9 %.

### Beispiel 6

Das Beispiel 1 wurde mit 2,5 g eines pulverförmigen 1 % Pt/Zeolith L-Katalysators bei 80°C wiederholt. Nach 6 h Reaktionszeit betrug die Ausbeute an 4-ADPA 36,9 %.

### Beispiel 7

Das Beispiel 1 wurde mit demselben Katalysator bei 40°C wiederholt. Nach 6 h Reaktionszeit betrug die Ausbeute an 4-ADPA 47,9 %.

### Beispiel 8

Das Beispiel 1 wurde mit demselben Katalysator unter Zugabe von 0,99 g (0,055 mol) Wasser bei 80°C wiederholt. Nach 1 h Reaktionszeit betrug die Ausbeute an 4-ADPA 32,6 %.

### Beispiel 9

Das Beispiel 1 wurde mit demselben Katalysator in 75 ml Toluol als Lösungsmittel bei 80°C wiederholt. Nach 6 h Reaktionszeit betrug die Ausbeute an 4-ADPA 17,3 %.

### Beispiel 10

Das Beispiel 1 wurde mit demselben Katalysator bei 25°C wiederholt. Nach 14 h Reaktionszeit betrug die Ausbeute an 4-ADPA 26,1 %.

### Beispiel 11

Das Beispiel 1 wurde mit 0,5 g eines pulverförmigen 5 % Pd/MgO-Katalysators (Katalysator A1) bei 80°C wiederholt. Nach 6 h Reaktionszeit betrug die Ausbeute an 4-ADPA 14,4 %.

### Beispiel 12

Das Beispiel 1 wurde mit 3,1 g (0,055 mol) KOH bei 80°C wiederholt. Nach 1,5 h Reaktionszeit betrug die Ausbeute an 4-ADPA 3,9 %.

### Beispiel 13

Das Beispiel 1 wurde mit 6,2 g (0,055 mol) KOt-C₄H₉ bei 80°C wiederholt. Nach 10 h Reaktionszeit betrug die Ausbeute an 4-ADPA 3,1 %.

### Beispiel 14

Das Beispiel 1 wurde mit 6,2 g (0,055 mol) KOt-C₄H₉ und 8,5 g (0,055 mol) (H₃C)₄NBr bei 80°C wiederholt. Nach 18 h Reaktionszeit betrug die Ausbeute an 4-ADPA 9,4 %.

### Beispiel 15

Das Beispiel 1 wurde mit 2,5 g (0,055 mol) NaOH bei 80°C wiederholt. Nach 1 h Reaktionszeit betrug die Ausbeute an 4-ADPA 1,0 %.

### Beispiel 16

Das Beispiel 1 wurde mit 0,5 g eines pulverförmigen 3,9 % Rh/Hydrotalcit-Katalysators (Katalysator A2) bei 80°C wiederholt. Nach 2 h Reaktionszeit betrug die Ausbeute an 4-ADPA 22,0 %.

### Beispiel 17

Das Beispiel 1 wurde mit 0,5 g eines pulverförmigen 3,8 % Pt/Hydrotalcit-Katalysators (Katalysator A3) wiederholt. Nach 2 h Reaktionszeit betrug die Ausbeute an 4-ADPA 28,3 %.

### Beispiel 18 (Vergleichsbeispiel)

Das Beispiel 1 wurde mit demselben Katalysator ohne Zusatz von Base bei 80°C wiederholt. Nach 14 h Reaktionszeit betrug die Ausbeute an 4-ADPA <0,3 %.

### Beispiel 19

Das Beispiel 1 wurde mit einer Mischung aus 1,18 g (0,011 mol) Nitrosobenzol und 5,42 g (0,044 mol) Nitrobenzol bei 80°C wiederholt. Nach 1 h Reaktionszeit betrug die Ausbeute an 4-ADPA 60,8 %.

### Beispiel 20

Das Beispiel 1 wurde mit einer Mischung aus 2,95 g (0,0275 mol) Nitrosobenzol und 3,39 g (0,0275 mol) Nitrobenzol bei 80°C wiederholt. Nach 1 h Reaktionszeit betrug die Ausbeute an 4-ADPA 35,2 %.

### Beispiel 21 (Druckreaktion)

In einem 21 Büchi-Glasautoklaven mit Vierfach-Propellerrührer wurden 133 g TMAOH. 2,3 H₂O, 960 ml Diethylenglykoldimethylether (Diglyme) und 2,4 g eines 5 % Pt/C-Katalysators (Katalysator B) auf 70°C erwärmt. Der Autoklav wurde mit Stickstoff inertisiert und 107,1 g Nitrosobenzol zugegeben. Anschließend wurde 4,4 Stunden mit 4 bar Wasserstoff und einer Rührerdrehzahl von 1000 U/min. bei 80°C hydriert.

Nach Ende der Reaktion wurde der Autoklav entspannt, mit Stickstoff gespült und bei 50°C 500 ml entionisiertes Wasser zugegeben. Zwei Phasen bildeten sich. Die Mischung wurd filtriert und die Phasen getrennt. Die organische Phase wurd viermal mit 300 ml entionisiertem Wasser ausgeschüttelt, sie war dann neutral. Die wäßrige Phase wurde dreimal mit 400 ml Toluol und 50 g NaCl ausgeschüttelt.

Die organischen Phasen wurden mit quantitativer HPLC analysiert, sie enthielten 48,4 g 4-ADPA (53,0 % bezogen auf Nitrosobenzol).

## Patentansprüche

1. Verfahren zur Herstellung von 4-Aminodiphenylamin, **dadurch gekennzeichnet, daß** man Nitrosobenzol oder Gemische aus Nitrosobenzol und Nitrobenzol mit Wasserstoff in Gegenwart von hydroxid-, oxid- und/oder alkoxidgruppenhaltigen Basen *und in Gegenwart von Metallen der 8. - 10. Gruppe des Periodensystems (nach IUPAC, neu) oder Kupfer und/oder Chrom, ggf. aufgebracht auf einen Katalysatorträger,* sowie in Gegenwart von inerten aprotischen Lösungsmitteln bei Temperaturen von 0 bis 200°C und Drücken von 0,1 bis 150 bar hydriert.

2. Verfahren nach Anspruch 1, **dadurch gekennzeichnet, daß** man bei Temperaturen von 25 bis 150°C und Drücken von 0,5 bis 70 bar hydriert.

3. Verfahren nach Anspruch 1, **dadurch gekennzeichnet, daß** man als hydroxid-, oxid- und/oder alkoxidgruppenhaltige Basen Alkalimetallhydroxide, Alkalimetalloxide, Erdalkalimetallhydroxide, Erdalkalimetalloxide, Alkalimetallalkoxide, Erdalkalimetallalkoxide sowie die entsprechenden Hydroxide, Alkoxide und Oxide der Elemente 58 bis 71 des Periodensystems der Elemente sowie quaternäre Alkylammoniumhydroxide einsetzt.

4. Verfahren nach Anspruch 1, **dadurch gekennzeichnet, daß** man die Basen in Mengen von 0,01 bis 3 Äquivalente pro Mol Nitrosobenzol einsetzt.

5. Verfahren nach Anspruch 1, **dadurch gekennzeichnet, daß** man als aprotische Lösungsmittel aromatische Kohlenwasserstoffe mit 6 bis 20 Kohlenstoffatomen, lineare oder cyclische Ether mit bis zu 5 Sauerstoffatomen und 2 bis 16 Kohlenstoffatomen, aromatische halogenierte Kohlenwasserstoffe mit 6 bis 20 Kohlenstoffatome sowie Amide mit 1 bis 10 Kohlenstoffatomen einsetzt.

6. Verfahren nach Anspruch 1, **dadurch gekennzeichnet, daß** man die inerten aprotischen Lösungsmittel in Mengen von 1 bis 99 Gew.-%, bezogen auf die Gesamtmenge der Reaktionsmischung, einsetzt.

7. Verfahren nach Anspruch 1, **dadurch gekennzeichnet, daß** man den Katalysator in diskontinuierlicher Fahrweise in Mengen von 0,01 bis 20 Gew.-%, bezogen auf eingesetztes Nitrosobenzol verwendet, oder in kontinuierlicher Fahrweise Belastungen von 0,01 bis 500 g Nitrosobenzol pro g Katalysator und Stunde einsetzt.

## Claims

1. Process for the preparation of 4-aminodiphenylamine, **characterised in that** nitrosobenzene or mixtures of nitrosobenzene and nitrobenzene are hydrogenated with hydrogen in the presence of bases containing hydroxide, oxide and/or alkoxide groups and in the presence of metals of Groups 8 to 10 of the Periodic Table (new IUPAC notation) or copper and/or chromium, optionally applied to a catalyst support, as well as in the presence of inert aprotic solvents at temperatures of from 0 to 200°C and at pressures of from 0.1 to 150 bar.

2. Process according to Claim 1, **characterised in that** hydrogenation is performed at temperatures of from 25 to 150°C and at pressures of from 0.5 to 70 bar.

3. Process according to Claim 1, **characterised in that** alkali metal hydroxides, alkali metal oxides, alkaline earth metal hydroxides, alkaline earth metal oxides, alkali metal alkoxides, alkaline earth metal alkoxides and the corresponding hydroxides, alkoxides and oxides of elements 58 to 71 of the Periodic Table of Elements and quaternary alkylammonium hydroxides are used as bases containing hydroxide, oxide and/or alkoxide groups.

4. Process according to Claim 1, **characterised in that** the bases are used in quantities of from 0.01 to 3 equivalents per mole nitrosobenzene.

5. Process according to Claim 1, **characterised in that** aromatic hydrocarbons having 6 to 20 carbon atoms, straight-chain or cyclic ethers having up to 5 oxygen atoms and 2 to 16 carbon atoms, aromatic halogenated hydrocarbons having 6 to 20 carbon atoms and amides having 1 to 10 carbon atoms are used as aprotic solvents.

6. Process according to Claim 1, **characterised in that** the inert aprotic solvents are used in quantities of from 1 to 99 wt.% in relation to the total quantity of reaction mixture.

7. Process according to Claim 1, **characterised in that** the catalyst is used in a batchwise operation in quantities of from 0.01 to 20 wt.% in relation to nitrosobenzene used, or in a continuous operation loadings of from 0.01 to 500 g nitrosobenzene are applied per gram of catalyst and per hour.

## Revendications

1. Procédé de préparation de 4-aminodiphénylamine, **caractérisé en ce que** l'on soumet du nitrosobenzène ou des mélanges de nitrosobenzène et de nitrobenzène à une hydrogénation avec de l'hydrogène en présence de bases contenant des groupes hydroxyde, oxyde et/ou alkylate et en présence de métaux des groupes 8 à 10 du système périodique (selon l'UICPA, nouveau) ou de cuivre et/ou de chrome, éventuellement déposés sur un support de catalyseur, et en présence de solvants aprotiques inertes à des températures de 0 à 200°C et sous des pressions de 0,1 à 150 bar.

2. Procédé selon la revendication 1, **caractérisé en ce que** l'on effectue l'hydrogénation à des températures de 25 à 150°C et sous des pressions de 0,5 à 70 bar.

3. Procédé selon la revendication 1, **caractérisé en ce que** l'on utilise comme bases contenant des groupes hydroxyde, oxyde et/ou alkylate des hydroxydes de métaux alcalins, des oxydes de métaux alcalins, des hydroxydes de métaux alcalino-terreux, des oxydes de métaux alcalino-terreux, des alkylates de métaux alcalins, des alkylates de métaux alcalino-terreux et les hydroxydes, alkylates et oxydes correspondants des éléments 58 à 71 du système périodique des éléments, ainsi que des hydroxydes d'alkylammonium quaternaire.

4. Procédé selon la revendication 1, **caractérisé en ce que** l'on utilise les bases en des quantités de 0,01 à 3 équivalents par mol de nitrosobenzène.

5. Procédé selon la revendication 1, **caractérisé en ce que** l'on utilise comme solvants aprotiques des hydrocarbures aromatiques de 6 à 20 atomes de carbone, des éthers linéaires ou cycliques ayant jusqu'à 5 atomes d'oxygène et de 2 à 16 atomes de carbone, des hydrocarbures aromatiques halogénés de 6 à 20 atomes de carbone et des amides de 1 à 10 atomes de carbone.

6. Procédé selon la revendication 1, **caractérisé en ce que** l'on utilise les solvants aprotiques inertes en des quantités de 1 à 99 % en masse par rapport à la quantité totale du mélange réactionnel.

7. Procédé selon la revendication 1, **caractérisé en ce que**, dans un mode opératoire discontinu, on utilise le catalyseur en des quantités de 0,01 à 20 % en masse par rapport au nitrosobenzène introduit, ou, dans un mode opératoire continu, on utilise des charges de 0,01 à 500 g de nitrosobenzène par g de catalyseur et par heure.
